(19) Europäisches Patentamt
European Patent Office
Office européen des brevets

(11) **EP 2 953 652 B1**

(12) **EUROPÄISCHE PATENTSCHRIFT**

(45) Veröffentlichungstag und Bekanntmachung des Hinweises auf die Patenterteilung:
**21.12.2016 Patentblatt 2016/51**

(21) Anmeldenummer: **14705309.4**

(22) Anmeldetag: **05.02.2014**

(51) Int Cl.:
*C11D 7/26* *(2006.01)*        *A01N 37/36* *(2006.01)*
*A01N 59/12* *(2006.01)*      *A61L 2/18* *(2006.01)*

(86) Internationale Anmeldenummer:
**PCT/EP2014/052270**

(87) Internationale Veröffentlichungsnummer:
**WO 2014/122187 (14.08.2014 Gazette 2014/33)**

(54) **BEHANDLUNGSFLÜSSIGKEIT ZUR REINIGUNG EINES IMPLANTAT-TEILS**

TREATMENT LIQUID FOR CLEANING AN IMPLANT PART

LIQUIDE DE TRAITEMENT CONÇU POUR NETTOYER UNE PARTIE D'IMPLANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.02.2013 DE 102013201883**

(43) Veröffentlichungstag der Anmeldung:
**16.12.2015 Patentblatt 2015/51**

(73) Patentinhaber: **Zyfoma GmbH**
**64331 Welterstadt (DE)**

(72) Erfinder:
• **BRODBECK, Urs**
**CH-8703 Erlenbach (CH)**
• **ZIPPRICH, Holger**
**64342 Malchen (DE)**

(74) Vertreter: **Walkenhorst, Andreas**
**Tergau & Walkenhorst**
**Patentanwälte - Rechtsanwälte**
**Eschersheimer Landstrasse 105-107**
**60322 Frankfurt (DE)**

(56) Entgegenhaltungen:
**WO-A1-2010/139762      JP-A- H08 299 999**
**JP-A- 2008 214 591      US-A- 3 420 760**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft eine Behandlungsflüssigkeit, insbesondere zur Reinigung von bakteriell verunreinigten Oberflächen von Knochenimplantaten, Dentalimplantaten oder anderen mit Biofilm verunreinigten Komponenten. Sie betrifft weiterhin die Verwendung der Behandlungsflüssigkeit, insbesondere zur Reinigung eines in den Kieferknochen eines Patienten eingebrachten Dental-Implantat-Teils.

Aus der nicht vorveröffentlichten deutschen Patentanmeldung mit dem Aktenzeichen 10 2012 022 593.8, ist ein Behandlungselement, insbesondere zur Verwendung mit einem Implantat-Teil, sowie ein Verfahren zum Reinigen eines Dental-Implantat-Teils bekannt. An der von Gewebe und Gewebeflüssigkeit umschlossenen festen Oberfläche von Implantaten kann sich nämlich ein Biofilm bilden, der von Bakterien besiedelt wird, die letztlich zu chronischen und wiederkehrenden Infektionen führen können. Dieses Erkrankungsbild wird als Periimplantitis bezeichnet. Insbesondere im dentalen Bereich ist ähnlich wie bei der Parodontitis eine Kombination aus vernachlässigter Mundhygiene, Anhaftung von Biofilm an der üblicherweise mikrorauen Oberfläche des Pfostenteils und weiterer Faktoren ursächlich für das Vollbild der Periimplantitis, welche sich durch eine zunehmende Belastung und Zerstörung des Hart- und Weichgewebes auszeichnet. Die Bereiche, an denen sich das Hart- und/oder Weichgewebe zurückzieht, werden dabei in der Regel mit einem Biofilm überzogen.

Das in der genannten Anmeldung beschriebene Reinigungsverfahren beruht auf dem Konzept, den die Verunreinigung bildenden Biofilm bzw. die Keime ausgehend von der Implantatoberfläche abzutöten und zu entfernen, ohne dabei die Implantatoberfläche zu beschädigen. Dazu ist ein elektrolytischer Prozess vorgesehen, bei dem Ionen (Kationen und/oder Anionen) mittels elektrostatischer Kräfte durch den Biofilm hindurch zu befördern. Diese Ionen reagieren an der Implantatoberfläche chemisch oder elektrochemisch. Durch diese Reaktionen werden neue Stoffverbindungen geschaffen und/oder die Ionen selbst und/oder Teile dieser Ionen in den atomaren Zustand überführt. Darüber hinaus besteht zudem die Möglichkeit, dass die Ionen mit dem Oberflächenmaterial reagieren (z. B. Bildung einer Oxidschicht oder Materialabtrag).

Die keimtötende Wirkung dieses Prozesses basiert auf unterschiedlichen Effekten. Zum einen werden durch das Anlegen einer elektrischen Spannung Ionen aus dem Biofilm selbst (auch aus den Bakterien) zur Anode oder Kathode befördert. Dies kann zur Abtötung von Bakterien und Viren führen. Darüber hinaus können die Ionen, während sie den Biofilm passieren, biochemische Reaktionen eingehen, was ebenfalls zur Abtötung von Bakterien und/oder Viren führen kann. Eine weitere Möglichkeit der Abtötung besteht darin, dass die an der Implantatoberfläche neu gebildeten Stoffverbindungen antibakterielle und/oder antivirale und/oder antifungizide Wirkung besitzen. Dies kann natürlich auch passieren, wenn die Ionen in den atomaren Zustand übergehen. Bei all den oben beschriebenen Prozessen besteht grundsätzlich die Gefahr, dass sich toxische, zellschädigende und/oder knochenschädigende Stoffe oder Stoffverbindungen bilden oder freigesetzt werden könnten.

Die WO2010139762 beschreibt ein Reinigungsverfahren für medizinische Implantate mit einer säurehaltigen Behandlungsflüssigkeit.

Der vorliegenden Erfindung liegt die Aufgabe zu Grunde, eine Behandlungsflüssigkeit anzugeben, die für die Verwendung in dem genannten Behandlungssystem besonders geeignet ist. Insbesondere soll eine Behandlungsflüssigkeit angegeben werden, die in besonderem Maße und mit hoher Wirksamkeit zur Behandlung oder Reinigung von Komponenten eines Dental-Implantatsystems, anderer Implantat-Teile wie beispielsweise allgemein von Knochenimplantaten oder gegebenenfalls auch anderer mit Biofilm überzogener Bauteile besonders geeignet ist.

Diese Aufgabe wird erfindungsgemäß gelöst, indem die Behandlungsflüssigkeit als Grundbestandteil von einer wässrigen Lösung einer Säure gebildet ist, die mit einem Metallsalz derart versehen ist, dass sich eine Leitfähigkeit von mindestens 30 mS/cm, vorzugsweise mindestens 75 mS/cm und besonders bevorzugt mindestens 150 mS/cm ergibt.

Vorteilhafte Ausgestaltungen der Erfindung sind Gegenstand der Unteransprüche. Weitere und/oder alternative vorteilhafte Ausgestaltungen der Erfindung ergeben sich auch aus der Figurenbeschreibung.

Die Erfindung geht dabei von der Überlegung aus, dass eine geeignete Behandlungsflüssigkeit einerseits gezielt auf die Nutzung und Begünstigung der auf elektrolytischer Reinigung beruhenden Effekte des genannten Systems ausgelegt sein sollte, wobei andererseits gerade im Hinblick auf eine denkbare Verwendung der Flüssigkeit auch für die Reinigung von Implantat-Teilen im inserierten Zustand die Bildung toxischer, zellschädigender und/oder knochenschädigender bzw. generell gesundheitsgefährdender Stoffe konsequent vermieden oder zumindest auf ein klinisch akzeptables Niveau reduziert gehalten werden sollte. Um dies zu ermöglichen, ist die Behandlungsflüssigkeit einerseits gezielt für eine Eignung als Elektrolyt für die genannten elektrochemischen Prozesse ausgestaltet, wobei insbesondere eine hierfür geeignet gewählte Leitfähigkeit vorgesehen ist. Andererseits ist die Behandlungsflüssigkeit aber auch hinsichtlich der Auswahl und Zusammensetzung ihrer Grundbestandteile derart gewählt, dass die in der Flüssigkeit vorliegenden Stoffe und auch die beim Ablauf der elektrochemischen Prozesse entstehenden Produkte die Keimabtötung und gegebenenfalls auch die mechanische Ablösung des Biofilms besonders begünstigen, ohne dabei im Gegenzug nennenswerte Schäden oder Gefährdungen für das Körper- oder Knochengewebe zu bedingen.

Vorteilhafterweise ist als Metallsalz ein Salz von Aluminium, eines Alkali- oder Erdalakali-Metalls, insbesondere ein Kalium-, Natrium-, Magnesium- oder Calciumsalz vorgesehen. Ganz besonders bevorzugt ist dabei ein Kalium- oder Natriumsalz. Weiterhin vorteilhaft ist als Salzbildner für das Metallsalz Chlor oder Jod vorgesehen. Die Säure ist hingegen besonders bevorzugt eine organische Säure, besonders bevorzugt eine $\alpha$-Hydroxylcarbonsäure, vorzugsweise Milchsäure, Zitronensäure, Essigsäure, Apfelsäure oder eine Kombination aus diesen Bestandteilen. Eine besonders vorteilhafte Zusammensetzung der Grundkomponenten ist dann gegeben, wenn - im Sinne einer pH-Wert-Pufferung zur bedarfsweisen Kompensation einer Hydroxid-Produktion - der pH-Wert der Behandlungsflüssigkeit kleiner als 5, vorzugsweise kleiner als 4, besonders bevorzugt etwa 2,7 bis 2,9, ist.

Besonders vorteilhaft und in eigenständig erfinderischer Weise wird die Behandlungsflüssigkeit zur Reinigung von bakteriell verunreinigten Oberflächen von Knochenimplantaten, Dentalimplantaten oder anderen mit Biofilm verunreinigten Komponenten, d. h. grundsätzlich zur Entfernung von Biofilm, verwendet.

Die mit der Erfindung erzielten Vorteile bestehen insbesondere darin, dass durch die Kombination der Grundbestandteile Metallsalz und Säure und die damit einhergehende elektrische Leitfähigkeit aufgrund der Ionendichte die erwünschten elektrolytischen oder elektrochemischen Prozesse bei der Reinigung besonders begünstigt werden können. Insbesondere kann durch die Säure in der Art einer pH-Wert-Pufferung einer unerwünschten Erhöhung des pH-Werts infolge der reaktionsbedingt eintretenden Erzeugung von Metall-Hydroxid entgegengewirkt werden.

Ein Ausführungsbeispiel der Erfindung wird anhand einer Zeichnung näher erläutert. Darin zeigen:

FIG. 1        ein zweiteiliges Dental-Implantatsystem in montiertem Zustand,

FIG. 2        das Dental-Implantatsystem gemäß FIG. 1 in Explosionsdarstellung,

FIG. 3        das Pfostenteil des Dental-Implantatsystems gemäß FIG. 1, 2 in seitlicher Ansicht,

FIG. 4        das Pfostenteil gemäß FIG. 3 im Längsschnitt,

FIG. 5        ein Behandlungselement für das Pfostenteil gemäß FIG. 3, 4 in perspektivischer Ansicht,

FIG. 6        das Behandlungselement gemäß FIG. 5 im Längsschnitt,

FIG. 7, 8    jeweils eine alternative Ausführungsform eines Behandlungselements in perspektivischer Ansicht,

FIG. 9        das Behandlungselement gemäß FIG. 8 im Längsschnitt, und

FIG. 10      ein Behandlungssystem.

Gleiche Teile sind in allen Figuren mit denselben Bezugszeichen versehen.

Die erfindungsgemäße Behandlungsflüssigkeit wird vorliegend anhand einer als bevorzugt angesehenen Verwendung in einem Behandlungssystem für ein Dentalimplantat näher erläutert, ist auf diese Verwendung aber in keiner Weise beschränkt. Vielmehr kommt analog ein Einsatz zur Behandlung oder Reinigung anderer Komponenten eines Dental-Implantatsystems, anderer Implantat-Teile wie beispielsweise allgemein Knochenimplantaten oder gegebenenfalls auch anderer mit Biofilm überzogener Bauteile oder Komponenten in Betracht.

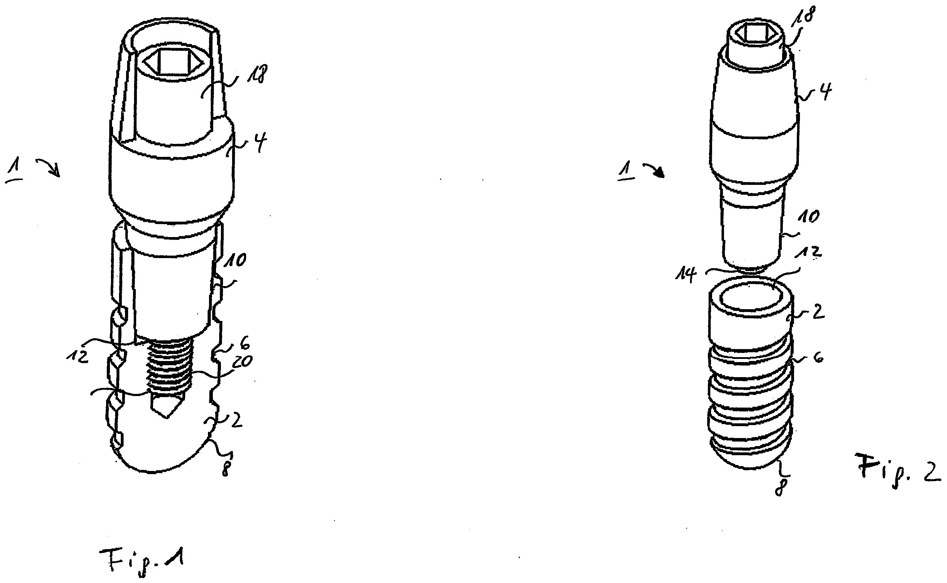

Das Dental-Implantatsystem 1 gemäß FIG. 1 ist zum Einsatz in den Kieferknochen anstelle eines extrahierten oder ausgefallenen Zahnes vorgesehen, um dort ein als Zahnersatz dienendes prothetisches Teil oder eine Krone zu halten. Das Dental-Implantatsystem 1 ist dabei mehrteilig ausgeführt und umfasst ein als so genanntes Pfostenteil ausgeführtes erstes Implantat-Teil 2 und ein diesem zugeordnetes, zur Anbringung eines Zahnersatzstücks vorgesehenes, auch als Aufbauteil oder Abutment bezeichnetes zweites Implantat-Teil 4. Das erste Implantat-Teil 2 oder Pfostenteil ist dabei außenseitig mit einem Außengewinde 6 versehen, das insbesondere am apikalen Ende 8 als selbstschneidendes Schraubengewinde ausgestaltet ist. Damit kann das erste Implantat-Teil 2 oder Pfostenteil durch Einschrauben an der vorgesehenen Stelle in den Kieferknochen eingesetzt werden.

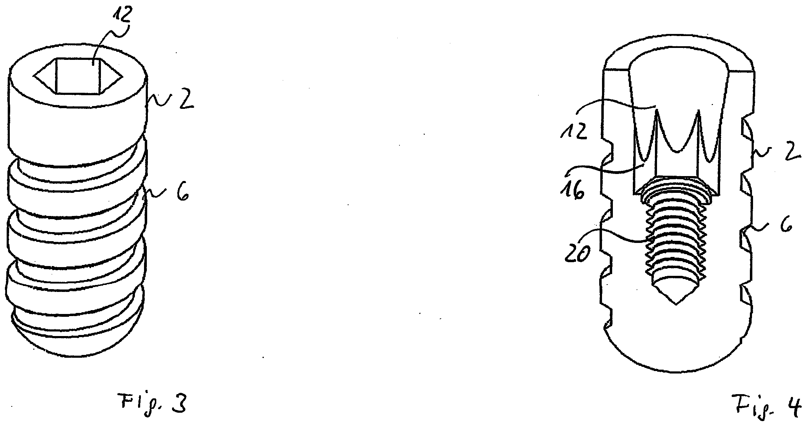

Um nach geeigneter Anbringung des Zahnersatzstücks oder der Prothese am Aufbauteil oder zweiten Implantat-Teil 4 ein Einbringen in das Pfostenteil oder erste Implantat-Teil 2 bei hoher mechanischer Stabilität zu ermöglichen, ist an das zweite Implantat-Teil 4 ein Verbindungszapfen 10 angeformt, der in einen zugeordneten, im ersten Implantat-Teil 2 vorgesehenen Aufnahmekanal 12 einschiebbar ist. Durch das Einschieben des Verbindungszapfens 10 in den Aufnahmekanal 12 entsteht eine mechanische Verbindung der Implantat-Teile 2, 4 miteinander. Für eine hohe mechanische Stabilität ist dabei der Verbindungszapfen 10 in seiner Außenkontur an die Innenkontur des Aufnahmekanals 12 angepasst, wobei beide in Längsrichtung gesehen konisch geformt sein können (Ausführungsbeispiel gemäß FIG. 2). Zudem kann, wie dies insbesondere im Ausführungsbeispiel gemäß FIG. 3 vorgesehen ist, die Außenkontur des Verbindungszapfens 10 - und dementsprechend angepasst die Innenkontur des Aufnahmekanals 12 - im Querschnitt mit einer mehrzähligen (im Ausführungsbeispiel sechszähligen) Symmetrie ausgestaltet sein, so dass beim Zusammenfügen der genannten Komponenten ein rotatorisches Gesperre entsteht und damit eine zuverlässige rotatorische Ausrichtung des Aufbauteils relativ zum Pfostenteil eingestellt werden kann. Im Ausführungsbeispiel gemäß FIG. 3, 4 ist zu diesem Zweck einer Indizierung oder zur Ausbildung eines rotatorischen Gesperres endseitig am Verbindungszapfen 10 ein Indizierungselement 14 mit seinerseits einem Querschnitt mit mehrzähliger Symmetrie angeordnet, das im montierten Zustand in ein entsprechendes, zugeordnetes Endkanalstück 16 im Aufnahmekanal 12 eingreift.

Das Dental-Implantatsystem 1 ist im Ausführungsbeispiel für eine Schraubverbindung der Implantat-Teile 2, 4 miteinander ausgeführt. Dazu ist jeweils eine Verbindungsschraube 18 vorgesehen, die in ein innerhalb des ersten Implantat-Teils 2 jeweils vorgesehenes Schraubgewinde 20 eingreift. Hinsichtlich ihrer Materialwahl sind die Implantat-Teile 2, 4 geeignet an den Einsatzzweck angepasst und grundsätzlich aus keramischem Material wie beispielsweise Zirkonoxid oder Aluminiumoxid oder auch aus einem geeignet gewählten Metall wie beispielsweise Titan gefertigt.

Generell besteht bei Dental-Implantatsystemen, insbesondere auch bei zweiteiligen Implantatsystemen der vorstehend beschriebenen Art, das Problem, dass durch ein Eindringen von Bakterien oder Keimen in den Gewebebereich in der Nähe der Insertionsstelle, insbesondere im Bereich des in den Kiefer eingebrachten Außengewindes 6, Entzündungen oder Entzündungsherde entstehen können. Derartige, insbesondere auch in Folge einer so genannten Periimplantitis entstehende Entzündungen können, insbesondere wenn sie sich über einen längeren Zeitraum entwickeln und verfestigen können, zu einer gravierenden Beeinträchtigung des Gewebes und des Knochens im Bereich der Insertionsstelle führen. Ohne geeignete Gegenmaßnahmen können diese Beeinträchtigungen dazu führen, dass das gesamte Implantatsystem, also insbesondere auch das bereits inserierte Pfostenteil oder zweite Implantat-Teil 4, wieder aus dem Knochen entfernt und durch andere Prothetik ersetzt werden muss. Dieser durch die Periimplantitis hervorgerufene, äußerst unerwünschte Effekt kann somit zu einem Totalverlust des Implantatsystems führen, so dass erneute chirurgische Maßnahmen wie beispielsweise ein Ausschaben des betroffenen Bereichs im Kieferknochen und die Neuversorgung mit einem Implantatsystem notwendig werden können. Durch eine derartige Entnahme kann es zudem zu Knochenverlust oder sonstigem Verlust an Gewebesubstanz kommen, die im Extremfall soweit führen kann, dass eine Neuversorgung mit einem anderen Implantat gar nicht mehr möglich ist. Eine derartige durch Periimplantitis hervorgerufene Notwendigkeit einer Neuversorgung kann auch nach vergleichsweise langen Zeiträumen nach dem ersten Einsetzen des Implantatsystems von beispielsweise bis zu einigen Jahren oder sogar Jahrzehnten auftreten.

Die im Zusammenhang mit einer Periimplantitis beobachteten Keime oder Bakterien können dabei grundsätzlich das Innere des Pfostenteils 2 besiedeln, haften aber in der Regel bevorzugt direkt an der Oberfläche des in den Kieferknochen inserierten Pfostenteils 2 im Kontaktbereich mit dem umgebenden Gewebe oder Knochenmaterial an, also insbesondere im Bereich des Außengewindes 6. In dessen Bereich kann die Oberfläche des Pfostenteils 2 mit einer Aufrauhung oder dergleichen versehen sein, um das Einwachsen in das Gewebe oder den Knochen besonders zu begünstigen und das Einheilen des Pfostenteils 2 nach der Insertion zu unterstützen. Gerade im Bereich einer derartigen, eigentlich als besonders günstig für das Implantatsystem angesehenen Aufrauhung der Oberfläche kann aber die Ansiedlung der Keime oder Bakterien vermehrt stattfinden, wobei die Rauigkeit ein gezieltes Entfernen der vorhandenen Keime oder Bakterien noch zusätzlich erschwert.

Es besteht daher der dringende Wunsch nach geeigneten Gegenmaßnahmen, um für den Fall einer sich anbahnenden oder bereits aufgetretenen Periimplantitis unter Erhaltung des bereits eingesetzten Implantatsystems, also insbesondere des bereits inserierten Pfostenteils 2, wirksam den Entzündungsherd bekämpfen und die eingedrungenen Keime abtöten zu können, so dass sich anschließend wieder gesundes Gewebe oder gesunde Knochensubstanz im Bereich um das Außengewinde 6 herum bilden kann. Dazu ist wünschenswert, zusätzlich zu einem gezielten Abtöten der Keime oder Bakterien im betroffenen Bereich auch noch deren Materialreste und Fragmente zuverlässig aus dem betroffenen Raumbereich zu entfernen, so dass anschließend der betroffene Bereich wieder von gesundem Gewebe oder Knochenmaterial ausgefüllt werden und sich wieder eine innige Verbindung zwischen der Außenoberfläche des Pfostenteils 2 und dem umgebenden Gewebe oder Knochenmaterial bilden kann. Zudem sollte der von der Bakterienbeschichtung gebildete Biofilm einschließlich der organischen Reste abgetöteter Bakterien zuverlässig entfernt werden.

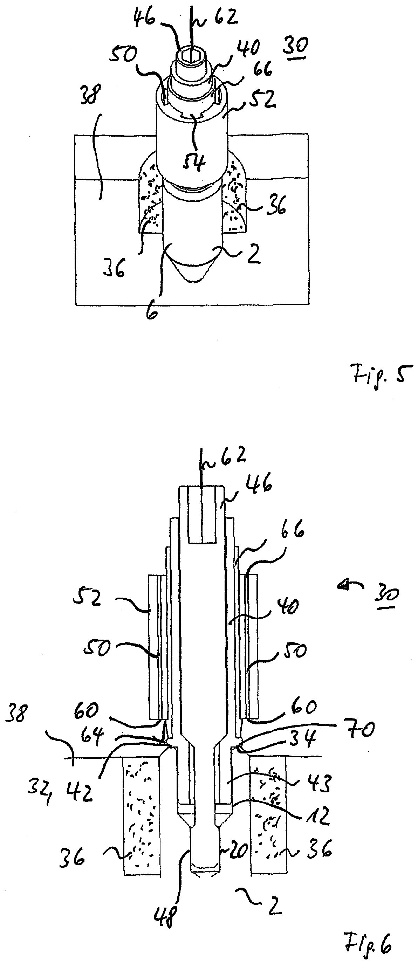

Zu diesem Zweck, also zum Abtöten von Keimen oder Bakterien im Insertionsbereich des Pfostenteils 2 und insbesondere auch zum anschließenden Ausspülen, Entfernen und Austragen der Gewebe- und Materialreste der abgetöteten Bakterien, ist ein Behandlungselement 30 vorgesehen, wie es in perspektivischer Ansicht in FIG. 5 und im Längsschnitt in FIG. 6 gezeigt ist. Das Behandlungselement 30 ist dabei im Ausführungsbeispiel aufgrund der an sich zweiteiligen Ausführung des Implantatsystems 1 in der Art eines Behandlungsabutments ausgeführt und zur Durchführung der genannten Behandlung für das dargestellte zweiteilig ausgeführte Implantatsystem 1 vorgesehen, wobei ein vorübergehendes Aufsetzen des Behandlungsabutments 30 auf das Pfostenteil 2 anstelle des eigentlichen Abutments oder zweiten Implantat-Teils 4 erfolgen soll. Die nachfolgenden Ausführungen beziehen sich daher auf diesen Fall eines zweiteiligen Implantatsystems 1; selbstverständlich kann aber auch in analoger Ausgestaltung eine entsprechende Verwendung für einteilige Implantate vorgesehen sein; hierfür müsste lediglich die mechanische Verbindung des Behandlungselements 30 mit dem auch während der Behandlung im Kieferknochen verbleibenden Teil des Implantatsystems geeignet ausgestaltet sein, beispielsweise über eine geeignete Kontaktfläche, mit der das Behandlungselement 30 anstelle der Prothetik auf das Abutment des Implantats aufgesetzt werden kann. Ebenso kann alternativ das Behandlungselement 30 oben auf das eigentliche Abutment 4 des Implantatsystems 1 aufgesetzt werden, so dass eine Verwendung beispielsweise zur Bekämpfung einer Entzündung des Weichgewebes (Mukositis) durch Abtötung der Bakterien und Reinigung der Oberfläche vorgesehen sein kann, ohne dass dazu das eigentliche Abutment 4 entfernt werden müsste.

Bei der im Ausführungsbeispiel vorgesehenen zweiteiligen Ausführung des Implantatsystems 1 wird zur Durchführung der nachfolgend näher beschriebenen Behandlung zunächst - gegebenenfalls nach Entfernen der am eigentlichen Abutment oder zweiten Implantat-Teil 4 angebrachten Prothetik - die Schraubverbindung zwischen erstem und zweitem Implantat-Teil 2, 4 gelöst und das zweite Implantat-Teil 4 abgenommen. Das erste Implantat-Teil oder Pfostenteil 2 verbleibt dabei im Kieferknochen. Anschließend wird das Behandlungsabutment 30 anstelle des eigentlichen Abutments 4 auf das Pfostenteil 2 aufgesetzt und über die Schraubverbindung mit diesem verbunden. Das Behandlungselement 30 weist dazu eine im Wesentlichen plane Kontaktfläche 32 auf, mit der es auf die Stirnkante 34 des Pfostenteils 2 aufgesetzt werden kann. Die Kontaktfläche 32 kann unter Umständen auch die Funktion einer Dichtfläche erfüllen und dementsprechend ausgestaltet sein; insbesondere kann sie hierzu konisch ausgeführt sein.

Das Behandlungsabutment 30 beruht hinsichtlich seiner Ausgestaltung und prinzipiellen Ausführung auf dem Grundkonzept, die im Insertionsbereich des Pfostenteils 2 vorhandenen Keime oder Bakterien durch gezielte Zuführung eines Reinigungs- oder Desinfektionsmittels gezielt abzutöten, wobei eventuell an der Oberfläche des Pfostenteils 2, insbesondere im Bereich Außengewindes 6, noch anhaftende Reste oder Fragmente von Keimen und/oder Bakterien durch geeignete Beaufschlagung mit Strom oder Stromstößen von der Außenoberfläche des Pfostenteils 2 abgelöst werden sollen, so dass sie anschließend ausgewaschen werden können.

In einem ersten, sowohl bezüglich der Ausgestaltung des Systems als auch bezüglich der vorgesehenen Verfahrensschritte des Behandlungsverfahrens eigenständig als erfinderisch angesehenen Aspekt ist das Behandlungselement 30 daher sowohl strukturell als auch funktional/konzeptionell dafür ausgelegt, eine Behandlungsflüssigkeit zum Abtöten der Keime oder Bakterien und/oder zum Reinigen des inserierten Implantat-Teils 2 gezielt in den Insertionsbereich des Pfostenteils 2, insbesondere den Bereich von dessen Außengewinde 6, einzuspeisen.

In einem zweiten, ebenfalls sowohl bezüglich der Ausgestaltung des Systems und der Auswahl und Zusammensetzung der Grundbestandteile der verwendeten Behandlungsflüssigkeit als auch bezüglich der vorgesehenen Verfahrensschritte des Behandlungsverfahrens eigenständig erfinderischen Aspekt ist das Behandlungselement 30 dafür ausgelegt, die abgetöteten Bakterien oder Keime bzw. deren Reste oder Fragmente zuverlässig von der Außenoberfläche des Pfostenteils 2 abzulösen, so dass sie anschließend ausgespült werden können und sich nachfolgend gesundes Gewebe oder Knochenmaterial an die Oberfläche des Pfostenteils 2 anlegen und dieses wieder vollständig in gesundes Gewebe oder Knochenmaterial einwachsen kann. Für die Ablösung der Bakterien oder Keime bzw. deren Resten oder Fragmenten von der Oberfläche ist deren Benetzung mit einer leitfähigen Behandlungsflüssigkeit unter Beaufschlagung mit Strom, gegebenenfalls in Form von gepulsten Stromstößen, vorgesehen. Wie sich nämlich ebenfalls völlig überraschend herausgestellt hat, scheint gerade diese Beaufschlagung mit Strom in Kombination mit geeignet gewählten Ionenkonzentrationen in der Behandlungsflüssigkeit besonders zuverlässig das Ablösen der Bakterien oder Keime bzw. von deren Fragmenten oder Resten von der darunterliegenden Oberfläche zu bewirken, selbst wenn diese aufgeraut ist und eigentlich das Anhaften organischen Materials aufgrund ihrer Oberflächenstruktur besonders begünstigt.

Dabei liegt die überraschende Erkenntnis zugrunde, dass die Beaufschlagung des Pfostenteils 2 mit Strom unter Verwendung einer geeignet gewählten Behandlungsflüssigkeit im Bereich der Außenoberfläche des Pfostenteils, also insbesondere im Bereich des Außengewindes 6, zu einer elektrolytischen Reaktion in der Behandlungsflüssigkeit und damit gegebenenfalls zur Erzeugung von Gasblasen in unmittelbarer Nähe zur Oberfläche führt. Durch diese Gasblasenbildung an der Oberfläche des Pfostenteils 2 werden die oberflächlich anhaftenden Bestandteile oder Fragmente der Keime oder Bakterien mit abgelöst und restlos entfernt, so dass sie keine Basis und keinen Nährboden für eine Neuansiedlung von Keimen in diesen Bereichen bilden können. Zurück bleibt eine von Keimen, Bakterien oder deren Bestandteilen oder Resten befreite, angeraute und poröse Oberfläche des Pfostenteils 2, die gut als Basis für eine zukünftige Integration in das nachwachsende Knochengewebe dienen kann. Die verbleibende Oberfläche kann dabei auch durch eine Titan-Oxidschicht gebildet sein, die auch bei einem Anodisieren der Oberfläche entstehen würde.

Eine besondere Begünstigung dieser im Sinne einer zuverlässigen Reinigung der Oberfläche erwünschten Ablösung der an der Oberflächen haftenden Biofolmbestandteile aus dem inserierten Pfostenteil 2 ist durch eine vorteilhafte besonders geeignete Verfahrensführung bei der Beaufschlagung mit Strom erreichbar. Diese kann derart vorgenommen werden, dass die infolge des Stromflusses im Bereich der inserierten Oberfläche stattfindende elektrolytische Gasblasenbildung besonders verstärkt wird. Hierbei kann das Pfostenteil 2 anodisch oder auch kathodisch geschaltet werden. Insbesondere bei einer zumindest vorübergehend kathodischen Beschaltung des Pfostenteils 2 entsteht elektrolytisch induziert Wasserstoffgas, das besonders wirksam zur Gasblasenbildung beiträgt. Bei anodischer Schaltung des Pfostenteils entstehen hingegen, abhängig von der Zusammensetzung der Behandlungsflüssigkeit, Chlorgas, Sauerstoff, Stickstoff; Kohlenmonoxid und/oder Kohlendioxid. Die dadurch gebildeten Gasblasen steigen in der umgebenden Flüssigkeit auf und erzeugen dadurch Mitnahmeeffekte, über die die genannten Oberflächenbestandteile mit abgetragen und nach außen hin abgefördert werden. Beispielsweise wurde völlig überraschend beobachtet, dass bei Verwendung einer positive Ionen enthaltenden Lösung, beispielsweise einer wässrigen Salzlösung, diese Ionen sich bei einer kathodischen Beschaltung des Pfostenteils 2 an diesem abscheiden und damit die Gasblasenbildung deutlich verstärken. Beispielsweise führt die Anwesenheit von Na$^+$-Ionen bei kathodischer Verschaltung des Pfostenteils 2 zu erheblicher Gasblasenbildung, da Na+ an der Kathode mit dem umgebenden Wasser zu NaOH reagiert und dabei Wasserstoff freisetzt.

In einem dritten, ebenfalls sowohl bezüglich der Ausgestaltung des Systems als auch bezüglich der vorgesehenen Verfahrensschritte des Behandlungsverfahrens eigenständig erfinderischen Aspekt ist das Behandlungselement 30 für eine besonders einfach und effizient gehaltene Kombination der genannten Aspekte ausgestaltet. Dabei liegt das Konzept zugrunde, dass sowohl die vorgesehene Zuführung der Reinigungsflüssigkeit als auch das gezielte Ablösen der Bakterien- und Keimreste und -fragmente durch Aufbringung der genannten Bestromung in einem gemeinsamen System und somit mit besonders einfach gehaltenen Mitteln erreicht werden kann.

Die erfindungsgemäße Behandlungsflüssigkeit ist im Hinblick auf diese Aspekte geeignet gewählt und zusammengesetzt. Die Auswahl und Zusammensetzung der Grundbestandteile der Behandlungsflüssigkeit ist dabei insbesondere im Hinblick auf die beabsichtigte Wirkungsweise, d. h. der Aufbringung eines elektrischen Stroms im Raumbereich der behandlungsbedürftigen Oberfläche, vorgenommen, wobei insbesondere sichergestellt ist, dass eine für diesen Zweck ausreichend hohe elektrische Leitfähigkeit in der Behandlungsflüssigkeit vorliegt. Diese soll insbesondere durch eine ausreichend hoch gewählte Ionendichte in der Behandlungsflüssigkeit sichergestellt werden. Dazu ist als ein Grundbestandteil der Behandlungsflüssigkeit ein Metallsalz vorgesehen, bevorzugt in wässriger Lösung. Dieses liefert die Ionen für den Stromtransport, und zudem können die nach der jeweiligen Elektrodenreaktion entstehenden Umsetzungsprodukte auch noch geeignete biochemische Wirkungen aufweisen. Durch die gezielte Wahl einer ausreichend hohen elektrischen Leitfähigkeit soll bei einer Durchführung des Reinigungsverfahrens am inserierten Implantat sichergestellt werden, dass der Stromfluss durch die Behandlungsflüssigkeit und damit durch die behandlungsbedürftigen Teile und Komponenten, nicht aber durch das Körpergewebe des Patienten erfolgt, so dass eine Gefährdung des Patienten durch einen ungewollten Stromfluss durch Weichgewebe, Knochen, Blut und/oder andere Körpermaterialien minimiert werden kann. Die elektrische Leitfähigkeit der Behandlungsflüssigkeit sollte dabei möglichst ein Vielfaches der elektrischen Leitfähigkeit von Blut, Knochen, Weichgewebe, Fettgewebe oder anderen Körpermaterialien aufweisen.

Demzufolge werden bei der Auswahl und Zusammensetzung der Grundbestandteile für die Behandlungsflüssigkeit insbesondere folgende Leitfähigkeitswerte berücksichtigt (die elektrische Leitfähigkeit σ wird dabei in der üblichen Einheit mS/cm angegeben):

| | |
|---|---|
| Haut: | 0,03 - 0.1 mS/cm |
| Knochen: | 0,06 - 0,2 mS/cm |
| Fettgewebe: | 0,20 - 1,0 mS/cm |
| Muskelgewebe: | 0,80 - 2,5 mS/cm |
| Blut: | ca. 6,7 mS/cm |
| andere Körperflüssigkeiten: | ca. 15 mS/cm |

Um das Gefährdungspotential für den Patienten geeignet gering zu halten und den Stromfluss auf die erwünschten Regionen zu begrenzen, sollte die elektrische Leitfähigkeit daher mindestens das Doppelte, bevorzugt das Fünffache, besonders bevorzugt das Zehnfache der Leitfähigkeit sonstiger Körperflüssigkeiten betragen. Daher sollte die elektrische Leitfähigkeit der Behandlungsflüssigkeit einen Wert von mindestens 30 mS/cm, vorzugsweise mindestens 75 mS/cm und besonders bevorzugt mindestens 150 mS/cm aufweisen. Im Vergleich zu Blut bedeutet dies, dass die elektrische Leitfähigkeit der Behandlungsflüssigkeit vorzugsweise mindestens etwa das Fünffache, bevorzugt mindestens etwas das Zehnfache und besonders bevorzugt mindestens etwa das Zwanzigfache der Leitfähigkeit von Blut beträgt. Messungen haben ergeben, dass beim Einsatz einer solchermaßen gewählten Behandlungsflüssigkeit die elektrische Spannung, der das Körpergewebe, das Blut, die Körperflüssigkeiten etc. ausgesetzt werden, geringer als 6 V, vorzugsweise geringer als 3 V, besonders bevorzugt kleiner als 1,5 V ist. Damit können Schädigungen für den Patienten aufgrund der gering gehaltenen Spannungen sicher ausgeschlossen werden. Zur Einhaltung einer solchen Leitfähigkeit ist insbesondere die Ionenkonzentration in der Behandlungsflüssigkeit und in den sie bildenden Grundbestandteilen ausreichend hoch gewählt; hierfür können Laugen, Säuren, Salze und/oder andere ionenbildende Stoffe oder Stoffverbindungen zum Einsatz kommen.

Bei der Auswahl und Zusammensetzung der Grundbestandteile der Behandlungsflüssigkeit ist in besonderem Maße berücksichtigt, dass die reinigende oder biofilmablösende Wirkung der elektrolytischen Behandlung einer kontaminierten Implantatoberfläche auf einer Kombination mehrerer Ursachen beruht, die möglichst ergänzend zueinander nutzbar gemacht werden sollten. Zum einen können sich beim Stromfluss durch den Elektrolyten bevorzugt im Bereich der Elektroden Gase oder Gasblasen bilden, welche eine abhebende (mechanische) Wirkung auf den Biofilm haben.

Die Entstehung dieser Gase erfolgt unmittelbar an der als Elektrode dienenden Implantatoberfläche und somit zwischen dieser und dem Biofilm. Die dabei entstehenden Gasblasen beeinflussen mit ihrer Wachstumsgeschwindigkeit und ihrer maximalen Größe den Ablösungsprozess.

Als zweite Ursache für die das Implantat reinigende oder den Biofilm ablösende Wirkung des elektrolytischen Prozesses ist die zersetzende, zerstörende und auflösende Wirkung der elektrolytisch entstehenden Stoffe oder Stoffverbindungen auf die eigentliche Anhaftung des Biofilms an der Implantatoberfläche, also auf den Klebe- oder Verankerungsmechanismus, zu nennen.

Die dritte Ursache für die reinigende oder ablösende Wirkung des elektrolytischen Prozesses basiert auf materialabtragenden Effekten des Implantatmaterials, wobei Bestandteile oder Partikel des eigentlichen Implantats in dessen Oberflächenbereich aus diesem herausgelöst werden.

Die vierte Ursache für die reinigende oder ablösende Wirkung des elektrolytischen Prozesses basiert auf der Oxidschichtbildung metallischer Implantate, welche dies erlauben. Hierbei durchdringen Metallatome des metallischen Grundmaterials die evtl. schon vorhandene Oxidschicht basierend auf der angelegten elektrischen Spannung und reagieren mit Stoffen des Elektrolyts (meist Sauerstoff => Metalloxidbildung). Bei Metallen, welche keine Oxidschicht bzw. keine mechanisch stabile Oxidschicht bilden, können auch nicht-oxydische Stoffverbindungen entstehen (meist Salze), die dann in Lösung gehen.

Die für die Bildung der Behandlungsflüssigkeit vorgesehenen Grundbestandteile sind im Hinblick auf diese Effekte geeignet gewählt und miteinander kombiniert. Zudem ist als grundlegendes Auslegungsziel berücksichtigt, dass keine toxischen oder anderweitig einen Patienten gefährdenden oder für ihn unangenehmen Effekte auftreten sollten, so dass die Behandlungsflüssigkeit auch für einen Einsatz am inserierten Dentalimplantat, also im Patientenmund, geeignet ist. Im Ausführungsbeispiel sind dabei als Grundbestandteile mindestens ein Salz einerseits und eine Säure andererseits, bevorzugt verdünnt mit Wasser, vorgesehen, deren Auswahl und Zusammensetzung sich insbesondere nach den genannten Kriterien richtet. Besonders bevorzugt ist dabei als Säure Phosphorsäure, Zitronensäure, Apfelsäure, Essigsäure, Milchsäure, Kohlensäure oder eine Kombination von diesen vorgesehen. Alternativ oder zusätzlich besonders bevorzugt ist dabei als Salz Natrium-, Calcium-, Aluminium-, Magnesium- oder Kaliumiodid, -chlorid, -nitrat, -carbonat oder -hydrogencarbonat und/oder Ammoniumchlorit, -nitrat oder -iodid oder eine Kombination von diesen vorgesehen.

Des Weiteren ist dabei berücksichtigt, dass der vorgesehene elektrolytische Prozess wahlweise mit anodischer oder mit kathodischer Schaltung des Pfostenteils geführt werden kann. Demzufolge wird im Folgenden in eine Anodenreaktion und eine Kathodenreaktion unterteilt.

Bei einer Anodenreaktion, also bei anodischer Schaltung des Pfostenteils 2, werden an der Anode die in der Behandlungsflüssigkeit vorhandenen Anionen generell durch den Entzug von Elektronen oxidiert. Dabei kann es zu einer unmittelbaren Reaktion mit dem Material kommen, insbesondere zur Bildung einer Oxidschicht und/oder eines Salzes mit dem Implantatmaterial. Knochenimplantate und dementsprechend auch das Pfostenteil 2 bestehen meist aus Titan, Zirkonium, Tantal oder aus Legierungen dieser Metalle. Darüber hinaus werden noch andere Metalle hinzulegiert. Diese Metalle oder Metalllegierungen weisen meist einen hohen Grad an Oxidschichtbildungen auf. Diese Oxidschichtbildung hat eine passivierende Wirkung auf die Oberfläche. Die Folge darauf ist die Unterbindung oder zumindest sehr starke Reduzierung der Anodenreaktion dieser Metalle oder Metalllegierungen. Da sich im Biofilm meist Stoffverbindungen mit Sauerstoff befinden, ist eine Unterbindung dieser Passivierung meist nicht möglich. Sollte das Pfostenteil anodisch geschaltet sein, so ist der ablösende reinigende Effekt somit meist auf die Oxidschichtbildung beschränkt. Bei höheren Betriebsspannungen von beispielsweise mehr als 10 V konnte bei umfangreichen Untersuchungen zwar aufgezeigt werden, dass ein materialabtragender Prozess möglich ist, dass dieser aber mit einer starken Wärmeentwicklung verbunden ist. Diese Wärmeentwicklung kann zur unerwünschten Nekrose des Knochens führen. Darüber hinaus verändert der damit einhergehende Materialabtrag auch in unerwünschter Weise die Eigenschaften der ursprünglichen Implantatoberfläche.

Als Ausnahme hierzu hat sich überraschend gezeigt, dass bei einem Basismaterial des Pfostenteils 2, bei dem Aluminium als Legierungsbestandteil vorhanden ist (beispielsweise bei Titan grade5, das etwa 6 % Aluminiumanteil und 4 % Vanadiumanteil aufweist) auch eine anodische Bestromung des Pfostenteils 2 möglich ist, ohne dass die Bildung einer Oxidschicht den Prozess zu sehr behindert. Hierdurch kann, je nach Zusammensetzung der Behandlungsflüssigkeit, Chlor- oder Iodgas oder auch $CO_2$ direkt an der Oberfläche des Pfostenteils 2 erzeugt und somit unmittelbar für die angestrebte Ablösung des Biofilms nutzbar gemacht werden. Für eine derartige Verfahrensführung ist das Behandlungselement 30 besonders vorteilhaft mit einer leitfähigen Oberflächenbeschichtung versehen, beispielsweise aus DLC ("diamond like carbon"), einem Metall, leitfähigem Kunststoff oder einer elektrisch leitfähigen Keramik.

Als besonders vorteilhaft hat sich dabei herausgestellt, dass bei einem Basismaterial Titan grade IV oder Titan grade V des Pfostenteils durch Zugabe von $CO_2$ in die Behandlungsflüssigkeit trotz sich bildender Oxidschicht bei anodischer Bestromung eine $CO_2$-Bildung CI-Bildung und/oder I-Bildung möglich ist, die einen länger andauernden Stromfluss ermöglicht.

Aus den genannten Gründen ist das Pfostenteil 2 bei der Behandlung mit der Behandlungsflüssigkeit jedoch im Allgemeinen bevorzugt kathodisch geschaltet. Dabei wandern positiv geladene Ionen (Kationen) zur Oberfläche des Pfostenteils 2. Dies können insbesondere $H^+$-Ionen, Metallionen oder langkettige Kohlenwasserstoffionen, z. B. aus ionischen Flüssigkeiten, sein. Das als Grundbestandteil für die Behandlungsflüssigkeit vorgesehene Salz ist dabei insbesondere gezielt im Hinblick auf die Eigenschaften der Kationen ausgewählt, die den genannten Prozess begünstigen oder überhaupt ermöglichen sollen. Zur Erzeugung einer möglichst hohen elektrischen Leitfähigkeit eignen sich insbesondere kleine Ionen ($H^+$-Ionen oder Metallkationen), die zudem in der Art eines weiteren besonders günstigen Effekts den gegebenenfalls vorhandenen Biofilm vergleichsweise leicht durchdringen können. $H^+$-Ionen werden an der durch das Pfostenteil 2 gebildeten Kathode zu elementarem Wasserstoff H reduziert. Dies erzeugt eine Blasenbildung.

Alkalimetalle, Erdalkalimetalle und/oder Aluminium reagieren an der Kathode mit dem umgebenden Wasser zu elementarem Wasserstoff und seinem Metallkationen und $OH^-$-Ionen. Dies bedeutet, dass sich Wasserstoffblasen und das Hydroxid des verwendeten Metallions bilden. Durch die Kombination dieser Komponenten ist somit neben der ablösenden Wirkung des entstehenden Wasserstoffs erreicht, dass das Metallhydroxid antibakteriell wirkt und einen verdünnenden oder auflösenden Einfluss auf den Biofilm bzw. dessen Adhäsionsmechanismus ausübt.

Um Unverträglichkeiten mit dem Körpergewebe zu vermeiden, sind als Metallkationen insbesondere die körpereigenen (z. B. Kalium- und/oder Natriumionen) besonders bevorzugt. Darüber hinaus eignen sich auch Calcium, Magnesium und/oder Aluminiumionen. Das als Grundbestandteil für die Behandlungsflüssigkeit vorgesehene Salz ist daher besonders bevorzugt ein Salz dieser Metalle, insbesondere da diese Metallkationen ohnehin nur in Form eines Salzes, z. B. in Wasser gelöst, zur Verfügung gestellt werden können.

Diese Metallsalze können Verbindungen der genannten Metalle mit einem geeigneten Salzbildner, beispielsweise mit Schwefel, Phosphor, Stickstoff, Fluor, Chlor, Jod, Brom, Kohlenwasserstoff, Sauerstoff, Bor oder anderen Nichtmetallen, sein. Der Salzbildner wird dabei vorteilhafterweise unter Berücksichtigung des Grundsatzes "je größer das Anion, desto geringer die elektrische Leitfähigkeit" und im Hinblick auf die grundsätzlich gewünschte hohe elektrische Leitfähigkeit geeignet gewählt. Als Anion werden zudem bevorzugt nur Stoffe in Betracht gezogen, welche weder die Gesundheit noch das periimplantäre Gewebe beeinflussen. Weiterhin gilt zu beachten, dass unangenehme Gerüche oder Geschmacksverbindungen unerwünscht sind. Aus diesen Gründen werden Schwefelanionen oder Anionen, die Schwefel in Kombination mit Sauerstoff oder anderen Elementen enthalten, als eher ungeeignet angesehen. Dies gilt auch für Fluor-, Brom-, Stickstoff- und Borionen, gegebenenfalls auch in Kombination mit weiteren Elementen.

Demgegenüber haben Phosphate, Phosphationen und Hydrogenphosphationen meist keine oder kaum eine schädliche Wirkung. Chlorionen oder Ionen, welche Chlor enthalten, haben meist eine antibakterielle Wirkung. Sollte das Chlorion allerdings elektrolytisch oxidiert werden und in Wasser elementar vorhanden sein, so bildet sich dabei Salzsäure und hypochlorige Säure. Dies würde zwar in Kombination mit dem kathodisch entstandenen Hydroxid zu einer Neutralisierung führen. Jedoch hat sich bei Untersuchungen gezeigt, dass das Chlor, welches an der Gegenelektrode zum Implantat (Anode) entsteht, in großem Maße dem Elektrolyten als Gas entweicht. Sollte das Chlor bei der Behandlung nicht restlos abgesaugt werden können, kann es zu starken Verätzungen in der Lunge und/oder den Schleimhäuten kommen. Hierbei gilt es abzuwägen, ob der Nutzen für den Patienten oder dessen Gefährdung größer ist.

Zu den Phosphaten des Aluminiums, des Kaliums, des Natriums, des Calciums oder des Magnesiums ist zudem zu bemerken, dass die Löslichkeit in Wasser so gering ist, dass eine ausreichende elektrische Leitfähigkeit des Elektrolyten nicht gewährleistet ist (diese Phosphate eignen sich allerdings sehr gut als Zusatzstoffe des Elektrolyten zur Pufferung des pH-Wertes). Chloride der vier eben aufgeführten Metalle hätten zwar eine ausreichende Löslichkeit in Wasser und eine gute Reinigungs- und Abtötungswirkung auf den Biofilm, können aber nicht als das Optimum angesehen werden. Bei Nitraten und/oder Nitriten ist eine Gefährdung des Patienten durch die Bildung von $NO_x$-Gasen zu erwarten. Aus diesem Grund ist vom Einsatz von Nitriten oder Nitraten abzuraten.

Im Hinblick auf die genannten Auslegungsziele, insbesondere für eine besonders gute Verträglichkeit für den Patienten, ist in bevorzugter Ausführung als Salzbildner Jod vorgesehen. Besonders vorteilhaft ist dabei, dass Jodsalze des Kaliums und des Natriums auch im menschlichen Körper von Natur aus vorhanden sind. Bei der Oxidation von Jodionen an der Anode entsteht zunächst elementares Jod, welches sich in eine Natriumiodid-/Kaliumiodidlösung lösen kann. Es entsteht dabei eine Jod-Kalium-Jodid-Lösung bzw. eine Jod-Natrium-Jodid-Lösung. Beide Lösungen sind starke Desinfektionsmittel, welche sich in der Humanmedizin bewährt haben.

Reine Natrium- oder Kaliumiodidlösungen oder ein Gemisch aus beiden haben jedoch als möglichen Nachteil die Natrium- und/oder Kaliumhydroxidbildung und den damit verbundenen pH-Wertanstieg zur Folge. Als problematisch könnte nämlich ganz allgemein bei der bereits oben beschriebenen Bildung von Metallhydroxid einzustufen sein, dass ein Metallhydroxid den pH-Wert des Elektrolyten erhöht. Ein solchermaßen erhöhter pH-Wert und die sich bildende Lauge oder Base des gelösten Metallhydroxids könnten einen unerwünschten Einfluss auf das umliegende Gewebe im Patientenmund und insbesondere den Knochen haben. Auch könnten umliegende Zähne geschädigt werden. Zudem könnte die Bildung von Hydroxiden dazu führen, dass diese sich aufgrund ihrer sehr geringen Wasserlöslichkeit auf dem Pfostenteil 2 oder allgemein auf dem behandlungsbedürftigen Bauteil niederschlagen und damit den weiteren Stromfluss und somit den Prozess insgesamt behindern. Allenfalls bei Verwendung eines Calciumsalzes in der Behandlungsflüssigkeit würde das entstehende Calciumhydroxid, das als Bestandteil des Knochenmaterials vorkommt, in den Knochen integriert werden können; Calcium ist somit besonders bevorzugter Bestandteil des Salzes. Um diese unerwünschten Einflüsse zu kompensieren, weist die Behandlungsflüssigkeit als weiteren Grundbestandteil in der Art eines pH-Puffers oder -Reduzierers die Säure auf.

Die Säure ist dabei ihrerseits gezielt in der Art eines Auslegungskriteriums derart gewählt, dass sie möglichst nicht den Patienten oder das periimplantäre Gewebe gefährdet, sondern zum einen das Hydroxid neutralisiert (und auch den pH-Wert möglichst nicht über 7 steigen lässt), wobei zum anderen die Reaktionsprodukte dem eigentlichen Ziel der Reinigung des Implantatkörpers und der Ablösung des Biofilms dienen sollten. Als Mineralsäuren kommen dazu bevorzugt Phosphorsäuren und/oder Phosphatsäuren in Frage. Diese sollten in ihrer Konzentration aus gesundheitsgefährdenden und/oder knochen-/gewebegefährdenden Gründen auf Höchstwerte von 30 % oder bevorzugt von 10 bis 20 % limitiert sein. Eine besonders bevorzugte Säure, welche auch als Mineralsäure angesehen wird und einen besonders positiven Effekt auf das Gesamtziel der Abtötung und Reinigung hat, ist hingegen die Kohlensäure. Diese ist jedoch in ihrer anwendbaren Menge durch das vergleichsweise geringe Lösungsvermögen in Wasser limitiert.

Organische Säuren stellen demgegenüber ähnlich wie Mineralsäuren pH-Wert-reduzierende und Hydroxid neutralisierende H$^+$-Ionen zur Verfügung. Da sie zudem keine oder allenfalls geringfügige Schädigungen im Gewebe erzeugen oder beim Patienten insgesamt hervorrufen, sind derartige organische Säuren als Grundbestandteil für die Behandlungsflüssigkeit ganz besonders bevorzugt. Organische Säuren wären z. B. Alkansäuren, Fruchtsäuren, Carbonsäuren sowie Hydroxylcarbonsäuren. Als besonders geeignete Säuren haben sich $\alpha$-Hydroxylcarbonsäuren herausgestellt. Insbesondere zeigen die besonders bevorzugten Säuren Milchsäure, Zitronensäure und Apfelsäure keinerlei gesundheitsschädigende Effekte auf den Patienten generell oder auf das periimplantäre Gewebe. Speziell bei stark mit Biofilm behafteten und kontaminierten Implantaten, auf welchen sich auch Zahnstein gebildet hat, zeigten bereits vergleichsweise geringe Dosierungen an Essigsäure einen guten Reinigungserfolg. Weitere Säuren, welche die Reinigung sowie den bakterientötenden Effekt aufweisen, aber aus gesundheitlichen Gründen nicht unbedenklich sind, wären die Fumarsäure, Gluconsäure, Glycolsäure, Salicylsäure, Mandelsäure, Weinsäure, Oxalsäure und Ameisensäure.

Bei der Neutralisierung des Hydroxidions OH- mit dem entsprechenden H$^+$-Ion einer Säure entsteht zusätzlich das Metallsalz der verwendeten Säure des entsprechenden Metallhydroxids. Der vorgesehene Einsatz der Säure ist somit nicht nur zur Pufferung des pH-Werts vorteilhaft, sondern trägt überdies noch zur Umwandlung des vergleichsweise schlecht wasserlöslichen Hydroxids in vergleichsweise gute wasserlösliche Salze bei und verhindert somit die Abscheidung unerwünschten und für den Prozess hinderlichen Niederschlags auf dem behandlungsbedürftigen Bauteil. Die genannten Salze werden insbesondere bei der Kombination der genannten bevorzugten Materielaien u. a. auch in der Medizin eingesetzt. Bei der Neutralisation des Kalium-, Natrium- und/oder Calciumhydroxids mit Milchsäure bildet sich das Kaliumlaktat (es besitzt eine breitbandige antimikrobielle Wirkung), Natriumlaktat bzw. Calciumlactat. Werden die sich bildenden Hydroxide hingegen mit Zitronensäure neutralisiert, bilden sich Zitrate des Kaliums, Natriums bzw. Calciums. Gerade für Natriumzitrat ist dies besonders vorteilhaft, da dieses die Blutgerinnung verhindert. Dies ist besonders vorteilhaft, da während des Prozesses austretendes, auf der Implantatoberfläche gerinnendes Blut die Ionenwanderung zur Implantatoberfläche und damit die Fortsetzung des Behandlungsprozesses insgesamt behindern könnte.

Bei der Neutralisation der Hydroxide mit Apfelsäure entstehen hingegen Malate des jeweiligen Kations, welche ebenfalls günstige Auswirkungen auf den Prozess haben. Bei der Neutralisation der Hydroxide mit Essigsäure entstehen Acetate des Kaliums, Natriums und/oder Calciums, welche ebenfalls einen günstigen Einfluss auf den Prozess haben.

Die Lactate, Zitrate, Malate und/oder Acetate des Kaliums, Natriums und/oder Calciums besitzen alle eine säureregulierende Wirkung und sind so verträglich, dass sie nach den jetzigen EU-Verordnungen für Lebensmittelzusatzstoffe in ihrer Verwendung an keine Mengenlimitierung gebunden sind.

Bei der Verwendung von Säuren in dem Elektrolyten in Kombination mit Jodiden und/oder Chloriden des Natriums, Kaliums, Magnesiums, Aluminiums und/oder Calciums hat sich bei der elektrolytischen Anwendung überraschend herausgestellt, dass durch die direkte Reduzierung des H$^+$-Ionen die Blasenbildung derart positiv beeinflusst wird, dass sich der Biofilm deutlich schneller und besser ablöst. Dabei entsteht mit hoher Erzeugungsrate eine Vielzahl vergleichsweise kleiner Bläschen, die aufgrund ihrer vergleichsweise geringen Größe den Biofilm als Ganzes und nicht nur lokal von der darunterliegenden Oberfläche lösen können. Dadurch wird der Biofilm bevorzugt als Ganzes oder in vergleichsweise großen, zusammenhängenden Stücken statt in einer Vielzahl kleinerer Fragmente abgehoben, was eine deutlich verbesserte Reinigungswirkung bedingt.

Anstelle von Metallkationen können auch Ammoniumkationen verwendet werden. Hierbei besteht allerdings die Gefahr, dass bei dem elektrolytischen Prozess andere Ammoniumverbindungen (z. B. Ammoniak) gebildet werden. Dies stellt eine Gefährdung des Patienten dar und zeigt sich auch durch einen sehr unangenehmen Geschmack und Geruch.

Es wurde bei Versuchen beobachtet, dass sich der Biofilm teilweise in sehr kleinen Fragmenten oder aber in größeren zusammenhängenden Teilen ablöst. Letzteres wird bevorzugt, da damit vergleichsweise großflächig sehr günstige Reinigungsergebnisse erzielt werden können. Untersuchungen haben weiterhin gezeigt, dass der Abtransport des gelösten Biofilms und/oder seiner Fragmente durch eine Schaumbildung an der Implantatoberfläche begünstigt wird. Es hat sich herausgestellt, dass es günstig ist, dass nach dem Einsatz eines Elektrolyten aus den beschriebenen Metallsalzen, Säuren und Wasser, die insbesondere für die Abtötung und Ablösung zuständig sind, ein zweiter Elektrolyt zum Einsatz kommt, welcher zusätzlich eine Schaumbildung im Bereich der Kathode aufweist. Eine solche Schaumbildung kann erreicht werden, indem dem Elektrolyten bevorzugt zusätzlich ein Stoff zugegeben wird, der mindestens drei $CH_2$-Kettenglieder oder mindestens ein $CH_2$-Kettenglied und mindestens eine Kohlenstoffringverbindung aufweist. Hierbei können z. B. Öl und/oder Chlorhexidin zum Einsatz kommen. Darüber hinaus können auch ionische Flüssigkeiten verwendet werden, welche vorzugsweise ein I-, Cl- und/oder OH--Ionen aufweisen. Da der organische Kationenanteil einer ionischen Flüssigkeit unter Umständen auf der Implantatoberfläche reduziert wird und dort verbleicht, ist es in einer besonders günstigen Ausführung möglich, Knochenwachstumsfaktoren an diesen Kationenanteil anzuhängen.

Werden Chloride und Jodide im richtigen Verhältnis gemischt, kann die störende Chlorgasbildung vermieden werden. An der Anode entsteht:

$$2J + 5Cl + 6H_2O \rightarrow 10HCl + 2HIO_3$$

Dies bedeutet, dass an der Anode Salzsäure sowie Jodsäure gebildet werden. Diese haben sicherlich eine starke antimikrobielle Wirkung und werden auch beim Zusammentreffen mit dem sich kathodisch bildenden Hydroxid wieder neutralisiert.

Eine ganz besonders bevorzugte Zusammensetzung der Behandlungsflüssigkeit, die im Laborversuch besonders günstige Reinigungseigenschaft zeigte, umfasst eine wässrige Lösung von Natriumiodid (NaI) oder Kaliumiodid (KI) in einem Mischungsverhältnis von mindestens 5, bevorzugt mindestens 10, besonders bevorzugt mindestens 20 g des Salzes pro 30 ml Flüssigkeit (d. h. Wasser, $H_2O$, gegebenenfalls mit $CO_2$ angereichert), durch Zugabe von Milchsäure reduziert auf einen pH-Wert von etwa 2,7 bis 2,9.

Bei der Prozessführung ist eine mittlere Stromdichte am Pfostenteil 2 bzw. am behandlungsbedürftigen Bauteil von mindestens 50 mA/cm², vorteilhafterweise von mindestens 100 mA/cm², besonders bevorzugt von mindestens 250 mA/cm², vorgesehen, wobei diese Stromdichte auf die äußere Oberfläche des Pfostenteils 2 (also ohne Berücksichtigung von oberflächenvergößernden Eigenschaften wie beispielsweise Rauigkeit oder Oberflächenstruktur) bezogen ist. Zur Ablösung des Biofilms hat sich eine durchschnittliche Stromdichte von 50 mA/cm² bis 300 mA/cm², vorteilhafterweise von 100 mA/cm² bis 200 mA/cm² als besonders günstig erwiesen. Zum Abtransport der Biofilmfragmente sollte die mittlere Stromdichte vorzugsweise auf den Bereich von 300 mA/cm² bis 5.000 mA/cm² oder besonders vorteilhaft von 1.000 mA/cm² bis 2.000 mA/cm² erhöht werden.

Mit der Zugabe von $H_2O_2$ wird der Sprudeleffekt an der Kathode stark gemindert oder unterbunden. Es kommt zu einer sehr starken $H_2O$-Bildung, die zum Spülen der Oberfläche genutzt werden kann.

Zur gezielten Zuführung der genannten, als eigenständig erfinderisch angesehenen Behandlungsflüssigkeit in den behandlungsbedürftigen Raumbereich am Pfostenteil 2 weist das Behandlungselement 30 einen konstruktiven Aufbau auf, wie er den Darstellungen in perspektivischer Ansicht gemäß FIG. 5 und im Längsschnitt gemäß FIG. 6 entnehmbar ist. Dort ist das Behandlungselement 30 jeweils im auf das Pfostenteil 2 aufmontierten Zustand dargestellt. Ebenfalls dargestellt ist dabei ein das Pfostenteil 2 im Bereich seines Außengewindes 6 ringförmig umgebender Raumbereich 36 im Kieferknochen 38, der von Periimplantitis befallen und dementsprechend mit Bakterien belastet ist.

Das Behandlungselement 30 weist einen im Wesentlichen als Zylindermantelkörper ausgeführten Grundkörper 40 auf, der mit seiner die Kontaktfläche 32 bildenden Stirnfläche 42 auf die obere Stirnfläche oder -kante 34 des Pfostenteils 2 aufgesetzt ist. Für eine erhöhte mechanische Stabilität ist zudem an den Grundkörper 40 ein Verbindungszapfen 43 angeformt, der hinsichtlich Kontur und Geometrieparameter an den Aufnahmekanal 12 im Pfostenteil 2 angepasst und in diesen einschiebbar ist.

Im Innenraum des Grundkörpers 40 und koaxial mit diesem ist ein zentraler Innenkanal 44 vorgesehen, in dem eine Verbindungsschraube 46 geführt ist. Die Verbindungsschraube 46 greift mit ihrem Schraubengewinde 48 in das innerhalb des Pfostenteils 2 vorgesehene Schraubgewinde 20 ein. Anders als die für die Verbindung des eigentlichen Abutments 4 mit dem Pfostenteil 2 vorgesehene Verbindungsschraube 18 ist die Verbindungsschraube 46 nicht für eine hohe mechanische Belastbarkeit und Langlebigkeit der hergestellten Schraubverbindung ausgelegt; vielmehr liegen für die Verbindungsschraube 46 andere Auslegungskriterien zugrunde. Dabei ist insbesondere der nachfolgend erläuterte Behandlungsablauf berücksichtigt, bei dem die Verbindungsschraube 46 und mit dieser das Pfostenteil 2 als Elektrode für die Strompulse dienen sollen. Demzufolge ist die Verbindungsschraube 46 aus elektrisch leitfähigem Material, insbesondere aus einem Metall wie beispielsweise Titan, gefertigt.

Das Behandlungselement 30 ist zur Zuführung der Behandlungsflüssigkeit, die unter anderem auch das Abtöten von Keimen oder Bakterien bewirken kann, in den Raumbereich 36 ausgelegt. Dazu ist der Grundkörper 40 mit einer Anzahl von Medienkanälen 50 versehen, die eingangsseitig mit einem Versorgungs- oder Bespeisungssystem für die Behandlungsflüssigkeit verbunden ist. Im Ausführungsbeispiel sind die Medienkanäle 50 dabei durch in einen den Grundkörper 40 umgebenden Ringkörper 52 eingebrachte Nuten 54 gebildet. Der Ringkörper 52 ist dabei auf den Grundkörper 40 aufgeschoben, so dass die Nuten 54 nach innen hin durch den Außenmantel des Grundkörpers 40 abgeschlossen sind und damit ein Kanalsystem aus den Medienkanälen 50 bilden. Alternativ könnten die Medienkanäle natürlich auch auf andere Weise direkt in den Grundkörper 40 eingebracht sein.

In unmittelbarer Nähe zum Kontaktbereich der Stirnfläche 42 des Grundkörpers 40 mit der Stirnkante 34 des Pfostenteils 2 weist das durch die Medienkanäle 50 gebildete Kanalsystem eine Anzahl von Auslassöffnungen 60 auf, von denen der Übersichtlichkeit halber in FIG. 6 lediglich zwei gezeigt sind. Jeder Medienkanal 50 ist dabei im Ausführungsbeispiel mit einer Auslassöffnung 60 versehen. In Querschnitt und Anzahl können die Auslassöffnungen 60 aber auch an individuelle Vorgaben angepasst sein. Beispielsweise könnte eine einzige Auslassöffnung vorgesehen sein, die beispielsweise einen vollständig umlaufenden Ringspalt zwischen der Stirnfläche 42 und der Stirnkante 34 bildet. Alternativ kann auch eine Mehrzahl von Auslassöffnungen 60 vorgesehen sein, die insbesondere in Umfangsrichtung des Grundkörpers 40 gesehen gleichmäßig um dessen Umfang herum angeordnet sein können. Das durch die Medienkanäle 50 gebildete Kanalsystem mündet mit seinen Auslassöffnungen 60 unmittelbar benachbart zur Stirnfläche 42 und damit unmittelbar oberhalb des Raumbereichs 36, so dass das aus den Auslassöffnungen 60 ausströmende Medium mehr oder weniger direkt in den darunterliegenden Raumbereich 36 gelangt. Durch diese für sich als eigenständig erfinderisch angesehene Ausgestaltung des Grundkörpers 40 bildet das Behandlungselement 30 somit ein Kanalsystem, mit dem die Behandlungsflüssigkeit gezielt und wirksam direkt in den behandlungsbedürftigen Raumbereich 36 eingebracht werden kann. Zusätzlich ist das Behandlungselement 30 noch als elektrisches System spezifisch ausgestaltet. Dabei ist als Auslegungsgrundsatz insbesondere vorgesehen, eine gepulste Beaufschlagung des in den Medienkanälen 50 geführten Mediums, insbesondere der darin geführten Behandlungsflüssigkeit, mit Strompulsen zu ermöglichen. Das Behandlungselement 30 ist dabei dafür ausgelegt, den zu Reinigungszwecken des inserierten Implantat-Teils 2 vorgesehenen Stromfluss gezielt lokalisiert im behandlungsbedürftigen Raumbereich 36 aufbringen zu können. Das Behandlungselement 30 ist dabei nach dem Auslegungsprinzip aufgebaut, dass der elektrische Strom dem inserierten Implantat-Teil 2 zugeführt und dieses als Elektrode verwendet werden kann. Dazu umfasst das Behandlungselement 30 ein erstes, einen elektrischen Strompfad bildendes und über die Verbindungsschraube 46 mit dem Implantat-Teil 2 elektrisch verbundenes Leitungselement 62, das seinerseits an eine geeignet gewählte Strom- oder Spannungsquelle anschließbar ist.

Zur Bildung eines Gegenpols oder der Gegenelektrode ist die Nutzung der elektrischen Leitfähigkeit der in den Medienkanälen 50 geführten Behandlungsflüssigkeit vorgesehen. Dazu ist der Innenraum der Medienkanäle 50 seinerseits elektrisch mit dem anderen Pol der Strom- oder Spannungsquelle verbunden. Damit bilden die Auslassöffnungen 60 der Medienkanäle 50 in elektrischer Hinsicht einen Kontakt 64 oder einen elektrischen Kontaktpunkt, über den der Stromfluss in das Implantat-Teil 2 oder vom Implantat-Teil 2 erfolgt. Durch diese Nutzung der in unmittelbarer Nachbarschaft zum behandlungsbedürftigen Raumbereich 36 positionierten Auslassöffnungen 60 als elektrischen Kontakt 64 ist erreicht, dass der zu Behandlungs- und Reinigungszwecken angelegte elektrische Strom durch die von den Bakterien befallene Oberflächenzone des inserierten Implantat-Teils 2 hindurch- und von dort aus weitgehend unmittelbar, also insbesondere ohne "Umwege" über weiteres Körpergewebe oder dergleichen, zur Kontaktfläche 64 bzw. zu dem Kontaktpunkt fließen kann. Die Medienkanäle 50 einschließlich der darin geführten, elektrisch leitfähigen Behandlungsflüssigkeit und den entsprechenden Anschlusselementen bildet somit im Ausführungsbeispiel ein zweites, einen elektrischen Strompfad zu dem endseitig angeordneten Kontakt 64 bildendes Leiterelement 66.

Alternativ könnte das zweite Leiterelement 66 aber auch in der Art einer "konventionellen" Elektrode, also insbesondere als elektrisch leitfähiges nadelartiges Element aus Metall, ausgeführt sein. Diese könnte insbesondere in einer Längsrichtung im Wesentlichen parallel zur Zentralachse des Grundkörpers 40 verschiebbar an diesem gelagert sein. Zur Bildung dieser Elektrode oder auch einer bedarfsweise zusätzlich vorgesehenen dritten Elektrode, die beispielsweise zur lokalen Erzeugung eines elektrischen Feldes beispielsweise zur Feldverstärkung vorgesehen sein kann, kann gegebenenfalls noch ein geeignet geformter weiterer Metallkörper 68 vorgesehen sein. Dabei kann das Behandlungselement 30 auch ohne die Medienkanäle ausgeführt sein, wobei die Gegenelektrode und damit der zweite Strompfad ausschließlich über den Metallkörper 68 gebildet sein kann. In diesem Fall wird der Kontakt 64 über die endseitig freie Fläche des jeweiligen Elektrodenkörpers gebildet.

Durch die Positionierung der Auslassöffnungen 60 und/oder der endseitigen Kontaktfläche 69 des Metallkörpers 68 ist zudem sichergestellt, dass die durch sie gebildete Kontaktfläche 64 des zweiten Leiterelement 66 in seitlicher Richtung gesehen in einem Abstand von mindestens 1 mm und von höchstens 10 mm von der zentralen Längsachse des Dental-Implantat-Teils 2 positioniert ist.

Der Grundkörper 40 des Behandlungselements 30 könnte aus isolierendem Material wie beispielsweise einer Keramik oder einem Kunststoff gebildet sein. Im Ausführungsbeispiel ist er jedoch aus Metall, nämlich aus Titan, gefertigt. Um dabei eine zuverlässige elektrische Isolierung der Komponenten gegeneinander zu gewährleisten, ist er an seiner die Kontaktfläche zum Dental-Implantat-Teil 2 bildenden Stirnfläche 42 mit einer isolierenden Beschichtung 70 versehen und somit elektrisch isoliert ausgeführt. Des Weiteren ist der Ringkörper 52 aus isolierendem Material wie beispielsweise einer Keramik ausgeführt.

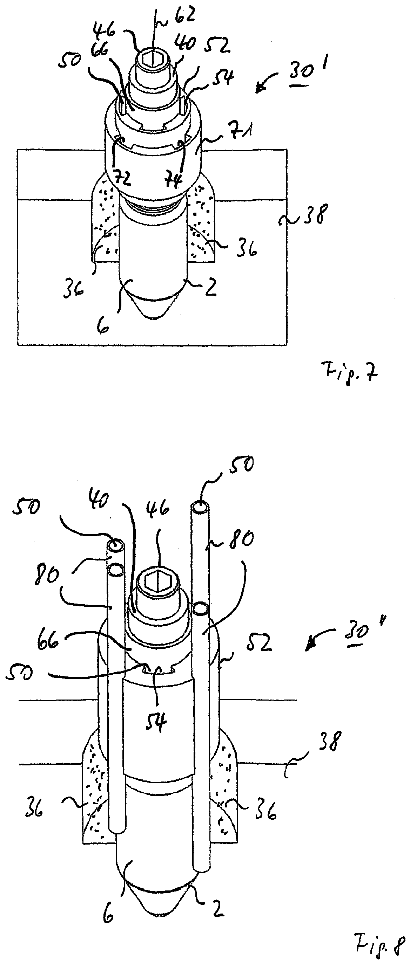

In einer alternativen Ausführungsform ist das Behandlungselement 30', wie dies in perspektivischer Ansicht in FIG. 7 gezeigt ist, mit einem weiteren Kanalsystem versehen, dass beispielsweise als Rückführkanal für die Behandlungsflüssigkeit, als separate Zuleitung zur Einbringung eines Mediengemischs oder auch als Absaugkanal vorgesehen sein kann. Dazu ist in dieser Ausführungsform der Ringkörper 52 von einem weiteren Ringkörper 71 umgeben, in den zur Bildung weiterer Medienkanäle 72 innenseitig ebenfalls Nuten 74 eingebracht sind.

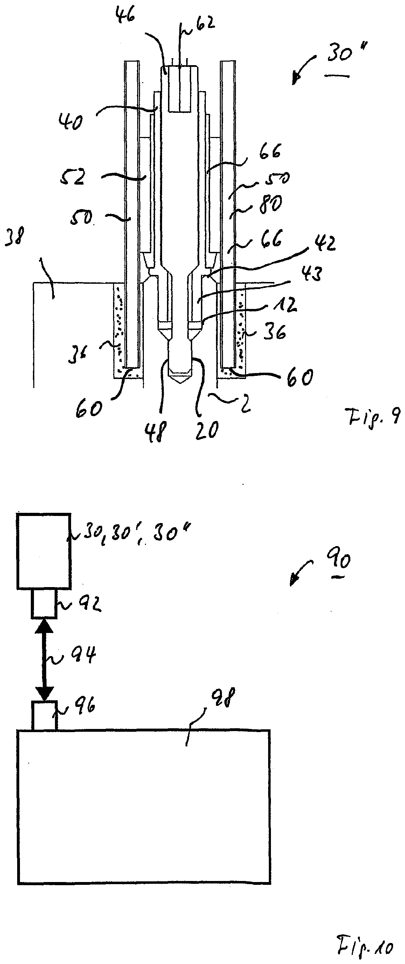

In den vorstehend erläuterten Ausführungsformen sind die Medienkanäle 50 und/oder die Leiterelemente 60, 66 in im Wesentlichen integrierter Bauweise ausgeführt und innerhalb des Grundkörpers 40 bzw. der mit diesem verbundenen Ringkörper 52, 71 geführt. Alternativ oder zusätzlich können aber auch einige oder alle der Medienkanäle 50 und/oder der Leiterelemente 60, 66 außerhalb am Grundkörper 40 angeordnet und über geeignete Haltesysteme mit diesem verbunden sein. Diese Ausführungsform ist im Ausführungsbeispiel in perspektivischer Ansicht gemäß FIG. 8 und im Längsschnitt gemäß FIG. 9 gezeigt. Zusätzlich zu den bereits erläuterten Komponenten ist das dort gezeigte Behandlungselement 30" mit außen am Ringkörper 52 angeordneten, in Längsrichtung verschiebbaren Kanalelementen 80 versehen. Diese können analog zu den Medienkanälen 50 in der Art von Kanülen oder dergleichen ausgeführt sein und mit der Behandlungsflüssigkeit beaufschlagt werden und zusätzlich als Leiterelement 66 dienen. Alternativ können sie aber auch metallisch in der Art von Elektroden ausgeführt sein und elektrisch geeignet mit der Strom- oder Spannungsquelle verbunden werden. Zusätzlich ist im Ausführungsbeispiel gemäß FIG. 8 noch eine Variante gezeigt, bei der zusätzlich zu den von den extern angeordneten Kanalelementen 80 gebildeten Medienkanälen auch noch durch Nuten 54 im Ringkörper 52 gebildete integrierte Medienkanäle 50 vorgesehen sind.

Das Behandlungselement 30, 30', 30" wird bevorzugt in einem Behandlungssystem 90 verwendet, wie es in FIG. 10 gezeigt ist. Das Behandlungssystem 90 ist für ein inseriertes Dental-Implantat-Teil oder Pfostenteil 2 vorgesehen und umfasst das Behandlungselement 30, 30', 30" und zusätzlich zu diesem ein Verbindungselement 92 zwischen diesem und einem Schlauchpaket 94, eine Steckverbindung 96 zwischen diesem und einer außerhalb des Patientenmundes angeordneten Versorgungs- und Steuereinheit 98. Diese Versorgungs- und Steuereinheit 98 beinhaltet eine elektrische Versorgung, welche zwischen der Elektrode im Pfostenteil 2 und einer weiteren Elektrode, welche sich in dem Behandlungselement 30, 30', 30", den Steckverbindungen 96, dem Schlauchpaket 94 und/oder der Versorgungs- und Steuereinheit 98 befinden kann, eine Spannung anlegen und/oder einen Strom fließen lassen kann.

Diese Spannung oder dieser Strom kann als Gleichspannung bzw. -strom, von der Polarität in beide Richtungen oder als Wechselspannung an die beiden Elektroden angelegt werden. Handelt es sich um eine Wechselspannung, kann diese als Sinus, Dreieck, Rechteck oder jede erdenkliche Überlagerung dieser mit unterschiedlichen Frequenzen sein. Darüber hinaus kann diese Wechselspannung von einer Gleichspannung überlagert sein. Es besteht auch die Möglichkeit, eine pulsierende Gleichspannung zu verwenden. Zur Erzeugung eines elektrischen Feldes kann eine dritte, elektrisch isolierte Elektrode vorzugsweise im Behandlungselement 30, 30', 30" angebracht sein.

Wie vorstehend beschrieben ist es besonders vorteilhaft, mehrere unterschiedlich zueinander zusammengesetzte Elektrolyte entweder nacheinander oder gleichzeitig an oder auf das Implantat zu bringen. Dazu ist das Behandlungssystem 90 geeignet ausgeführt. Insbesondere beinhaltet die Versorgungs- und Steuereinheit 98 Vorratsbehälter für mindestens zwei Flüssigkeiten oder Elektrolyte. Diese können über Pumpen und über eine oder mehrere Ventile oder Ventileinheiten gleichzeitig (mischend) oder nacheinander über das Schlauchpaket 94 in das Behandlungselement 30, 30', 30" gefördert werden. In einem besonders günstigen Fall beinhaltet die Versorgungs- und Steuereinheit 98 auch eine Absaugung, um die über das Behandlungselement 30, 30', 30" zugeführten Flüssigkeiten oder Elektrolyte nach ihrem Einsatz wieder absaugen zu können. Die Versorgungs- und Steuereinheit 98 beinhaltet in einer besonders günstigen Ausführung zudem eine $CO_2$-Aufbereit für Wasser oder andere Flüssigkeiten/Elektrolyte. Zur Prozessoptimierung kann in die Versorgungs- und Steuereinheit 98 auch eine Medientemperierung integriert sein.

Das Schlauchpaket 94 und die Steckverbindungen 96 sind so ausgelegt, dass sie den Stromfluss und den Medienfluss gewährleisten können. Bei einer vollständigen Ausstattung wären das insbesondere drei elektrische Leiter und zwei Flüssigkeits-/Elektrolytleiter.

Das Elektrodenmaterial kann aus dem gleichen Material wie das Pfostenteil 2 sein. Da die Pfostenteile 2 bevorzugt aus Titan oder einer Titanlegierung hergestellt sind, ist es bevorzugt, die weitere/weiteren Elektroden aus einem anderen Metall auszuführen. Titan und dem Titan ähnliche Metalle bilden bei der anodischen Bestromung meist eine schützende Oxidschicht, welche als Isolator fungiert. Um bei kathodischer Bestromung des Pfostenteils 2 nicht über eine solche Oxidschicht den Stromfluss einzuschränken, ist es vorteilhaft, ein nicht oder kaum eine Oxidschicht bildendes Metall als Gegenelektrode zu verwenden. In einem besonders günstigen Fall korrodiert diese Elektrode weder bei Kontakt mit den Medien/Elektrolyten noch bei der Beaufschlagung mit einer Spannung oder einem Strom. Vorzugsweise ist diese Elektrode aus Gold, Platin, Palladium ausgeführt.

Sollte auch das Innere des inserierten Implantates/Pfostenteils 2 kontaminiert sein und folglich gereinigt werden, besteht die Möglichkeit das Innere separat oder gemeinsam mit dem Medium zu spülen und mit Strom zu beaufschlagen.

Die Leiterelemente können auch in Form einer flexiblen oder festen Membrane ausgestaltet seine, welche keine Flüssigkeiten passieren lässt sondern lediglich in dem Elektrolyten vorhandene Ionen. Bei einer solchen Ausgestaltung mündet vorzugsweise einer der Strompfade im Inneren des Pfostenteils 2 und führt weiter an den in diesem Fall gar nicht oder nur teilweise abdichtenden Kontaktflächen 32 vorbei bis an die äußere Oberfläche des Pfostenteils 2.

Bezugszeichenliste

| 1 | Dental-Implantatsystem |
|---|---|
| 2 | erstes Implantat-Teil/Pfostenteil |
| 4 | zweites Implantat-Teil |
| 6 | Außengewinde |
| 8 | apikales Ende |
| 10 | Verbindungszapfen |
| 12 | Aufnahmekanal |
| 14 | Indizierungselement |
| 16 | Endkanalstück |
| 18 | Verbindungsschraube |
| 20 | Schraubgewinde |
| 30, 30', 30" | Behandlungselement/Behandlungsabutment |
| 32 | Kontaktfläche |
| 34 | Stirnkante |
| 36 | Raumbereich |
| 40 | Grundkörper |
| 42 | Stirnfläche |
| 43 | Verbindungszapfen |
| 44 | Führungshülse |
| 45 | Distanzstücke |
| 46 | Verbindungsschraube |
| 48 | Schraubengewinde |
| 50 | Kanal/Medienkanal |
| 52 | Ringkörper |
| 60 | Auslassöffnung |
| 62 | Leitungselement |
| 64 | Kontakt |
| 66 | Leiterelement |
| 68 | Metallkörper |
| 69 | Kontaktfläche |
| 70 | isolierende Beschichtung |
| 71 | Ringkörper |
| 72 | Medienkanal |
| 74 | Nut |
| 90 | Behandlungssystem |
| 92 | Verbindungselement |
| 94 | Schlauchpaket |
| 96, 98 | Versorgungs- und Steuereinheit |

**Patentansprüche**

1. Verwendung einer Behandlungsflüssigkeit, gebildet durch eine wässrige Lösung einer Säure, die mit einem Metallsalz derart versehen ist, dass sich eine Leitfähigkeit von mindestens 30 mS/cm ergibt, zur Entfernung von Biofilm von einem Bauteil, insbesondere einem Knochen- oder Dentalimplantat.

2. Verwendung nach Anspruch 1, wobei die Leitfähigkeit der Behandlungsflüssigkeit mindestens 75 mS/cm, besonders bevorzugt mindestens 150 mS/cm, beträgt.

3. Verwendung nach Anspruch 1 oder 2, wobei für die Behandlungsflüssigkeit als Metallsalz ein Salz von Aluminium, eines Alkali- oder Erdalakali-Metalls vorgesehen ist.

4. Verwendung nach einem der Ansprüche 1 bis 3, wobei für die Behandlungsflüssigkeit als Metallsalz ein Kalium-, Natrium-, Calcium-, Magnesium- oder Aluminiumsalz vorgesehen ist.

**EP 2 953 652 B1**

5. Verwendung nach einem der Ansprüche 1 bis 4, wobei für die Behandlungsflüssigkeit als Salzbildner für das Metallsalz Jod vorgesehen ist.

6. Verwendung nach einem der Ansprüche 1 bis 5, wobei für die Behandlungsflüssigkeit als Säure eine organische Säure vorgesehen ist.

7. Verwendung nach einem der Ansprüche 1 bis 6, wobei für die Behandlungsflüssigkeit als Säure eine $\alpha$-Hydroxylcarbonsäure, vorzugsweise Milchsäure, Zitronensäure, Essigsäure oder Apfelsäure oder eine Kombination aus diesen Bestandteilen vorgesehen ist.

8. Verwendung nach einem der Ansprüche 1 bis 7, bei der die Behandlungsflüssigkeit einen pH-Wert von weniger als 5, vorzugsweise von weniger als 4, besonders bevorzugt von etwa 2,7 bis 2,9, aufweist.

9. Behandlungsflüssigkeit zur Reinigung von bakteriell verunreinigten Oberflächen von Knochenimplantaten, Dentalimplantaten oder anderen mit Biofilm verunreinigten Komponenten, mit einer wässrigen Lösung eines Phosphats von Kalium, Natrium, Calcium, Magnesium oder Aluminium und/oder einer Säure, wobei die wässrige Lösung mit einem Metallsalz, gebildet aus Jod als Salzbildner, derart versehen ist, dass sich eine Leitfähigkeit von mindestens 30 mS/cm ergibt.

10. Behandlungsflüssigkeit nach Anspruch 9, deren Leitfähigkeit mindestens 75 mS/cm, besonders bevorzugt mindestens 150 mS/cm, beträgt.

11. Behandlungsflüssigkeit nach Anspruch 9 oder 10, bei der als Metallsalz ein Salz von Aluminium, eines Alkali- oder Erdalakali-Metalls vorgesehen ist.

12. Behandlungsflüssigkeit nach Anspruch 9 oder 10, bei der als Metallsalz ein Kalium-, Natrium-, Calcium-, Magnesium- oder Aluminiumsalz vorgesehen ist.

13. Behandlungsflüssigkeit nach einem der Ansprüche 9 bis 12, deren Säure eine organische Säure ist.

14. Behandlungsflüssigkeit nach einem der Ansprüche 9 bis 13, deren Säure eine $\alpha$-Hydroxylcarbonsäure, vorzugsweise Milchsäure, Zitronensäure, Essigsäure oder Äpfelsäure, oder eine Kombination aus diesen Bestandteilen ist.

15. Behandlungsflüssigkeit nach einem der Ansprüche 9 bis 14, die einen pH-Wert von weniger als 5, vorzugsweise von weniger als 4, besonders bevorzugt von etwa 2,7 bis 2,9, aufweist.

**Claims**

1. A use of a treatment liquid formed by an aqueous solution of an acid to which a metallic salt is added in such a manner that a conductivity of at least 30 mS/cm results, to remove a biofilm from a component, in particular a bone or dental implant.

2. The use of claim 1, wherein the conductivity of the treatment liquid is at least 75 mS/cm, particularly preferably at least 150 mS/cm.

3. The use of claim 1 or 2, wherein a salt of aluminium, of an alkali metal or of an alkaline earth metal is provided as metallic salt for the treatment liquid.

4. The use of any of claims 1 to 3, wherein a potassium, sodium, calcium, magnesium, or aluminium salt is provided as metallic salt for the treatment liquid.

5. The use of any of claims 1 to 4, wherein iodine is provided as a halogen for the metallic salt, for the treatment liquid.

6. The use of any of claims 1 to 5, wherein an organic acid is provided as an acid for the treatment liquid.

7. The use of any of claims 1 to 6, wherein an $\alpha$-hydroxy carbonic acid, preferably lactic acid, citric acid, ethanoic acid or malic acid, or a combination of these constituents, is provided as an acid for the treatment liquid.

8. The use of any of claims 1 to 7, wherein the treatment liquid has a pH-value of less than 5, preferably of less than 4, particularly preferably of approx. 2.7 to 2.9.

9. A treatment liquid for cleaning bacterially contaminated surfaces of bone implants, dental implants or other components contaminated with a biofilm, with an aqueous solution of a phosphate of potassium, sodium, calcium, magnesium, or aluminium, and/or of an acid, a metallic salt formed by iodine as a halogen being added to said aqueous solution in such a manner that a conductivity of at least 30 mS/cm, results.

10. The treatment liquid of claim 9, whose conductivity is at least 75 mS/cm, particularly preferably at least 150 mS/cm.

11. The treatment liquid of claim 9 or 10, wherein a salt of aluminium, of an alkali metal or of an alkaline earth metal is provided as metallic salt.

12. The treatment liquid of claim 9 or 10, wherein a potassium, sodium, calcium, magnesium, or aluminium salt is provided as metallic salt.

13. The treatment liquid of any of claims 9 to 12, whose acid is an organic acid.

14. The treatment liquid of any of claims 9 to 13, whose acid is an $\alpha$-hydroxy carbonic acid, preferably lactic acid, citric acid, ethanoic acid or malic acid, or a combination of these constituents.

15. The treatment liquid of any of claims 9 to 14, which has a pH-value of less than 5, preferably of less than 4, particularly preferably of approx. 2.7 to 2.9.

## Revendications

1. Utilisation d'un liquide de traitement formé par une solution aqueuse d'un acide à laquelle a été ajouté un sel métallique, de façon qu'une conductivité d'au moins 30 mS/cm résulte, pour enlever un biofilm d'un composant, en particulier d'un implant osseux ou d'un implant dentaire.

2. Utilisation selon la revendication 1, dans laquelle la conductivité du liquide de traitement est au moins 75 mS/cm, de préférence particulière, au moins 150 mS/cm.

3. Utilisation selon la revendication 1 ou 2, dans laquelle un sel d'aluminium, d'un métal alcalin ou d'un métal alcalinoterreux est prévu comme sel métallique pour le liquide de traitement.

4. Utilisation selon l'une quelconque des revendications 1 à 3, dans laquelle un sel de potassium, de sodium, de calcium, de magnésium ou d'aluminium est prévu comme sel métallique pour le liquide de traitement.

5. Utilisation selon l'une quelconque des revendications 1 à 4, dans laquelle iode est prévu comme halogène pour le sel métallique, pour le liquide de traitement.

6. Utilisation selon l'une quelconque des revendications 1 à 5, dans laquelle un acide organique est prévu comme acide pour le liquide de traitement.

7. Utilisation selon l'une quelconque des revendications 1 à 6, dans laquelle un acide carboxylique d'hydroxyle $\alpha$, de préférence acide lactique, acide citrique, acide acétique ou acide malique, ou une combinaison de ces constituants, est prévu comme acide pour le liquide de traitement.

8. Utilisation selon l'une quelconque des revendications 1 à 7, dans laquelle le liquide de traitement a une valeur pH de moins de 5, de préférence de moins de 4, de préférence particulière d'environ 2,7 à 2,9.

9. Liquide de traitement conçu pour nettoyer les surfaces bactériellement contaminées d'implants osseux, d'implants dentaires ou d'autres composants contaminés par une biofilm, avec une solution aqueuse d'un phosphate de potassium, de sodium, de calcium, de magnésium ou d'aluminium, et/ou d'un acide, un sel métallique formé par de l'iode comme halogène étant ajouté à dite solution aqueuse, de façon qu'une conductivité d'au moins 30 mS/cm résulte.

**10.** Liquide de traitement selon la revendication 9, dont la conductivité est au moins 75 mS/cm, de préférence particulière au moins 150 mS/cm.

**11.** Liquide de traitement selon la revendication 9 ou 10, dans laquelle un sel d'aluminium, d'un métal alcalin ou d'un métal alcalinoterreux est prévu comme sel métallique.

**12.** Liquide de traitement selon la revendication 9 ou 10, dans laquelle un sel de potassium, de sodium, de calcium, de magnésium ou d'aluminium est prévu comme sel métallique.

**13.** Liquide de traitement selon l'une quelconque des revendications 9 à 12, dont l'acide est un acide organique.

**14.** Liquide de traitement selon l'une quelconque des revendications 9 à 13, dont l'acide un acide carboxylique d'hydroxyle a, de préférence acide lactique, acide citrique, acide acétique ou acide malique, ou une combinaison de ces constituants.

**15.** Liquide de traitement selon l'une quelconque des revendications 9 à 14, qui a une valeur pH de moins de 5, de préférence de moins de 4, de préférence particulière d'environ 2,7 à 2,9.

Fig. 2

Fig. 1

Fig. 3

Fig. 4

EP 2 953 652 B1

Fig. 5

Fig. 6

Fig. 7

Fig. 8

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102012022593

- WO 2010139762 A